# EUROPEAN PATENT SPECIFICATION

(11) **EP 1 934 531 B1**
(45) Date of publication and mention of the grant of the patent: **20.02.2013**
(21) Application number: 06798891.5
(22) Date of filing: 25.09.2006
(51) Int. Cl.: A23L 3/28, H05B 6/64, H05B 6/80, A61L 2/10

(54) **STERILIZING DEVICE WITH ULTRAVIOLET RAY AND MICROWAVE OVEN HAVING THE SAME**
STERILISIERVORRICHTUNG MIT ULTRAVIOLETTSTRAHLUNG UND DAMIT AUSGESTATTETER MIKROWELLENHERD
DISPOSITIF DE STÉRILISATION À RAYONS ULTRAVIOLETS ET FOUR À MICRO-ONDES LE COMPORTANT

(30) Priority: 23.09.2005 KR 20050088909; 23.09.2005 KR 20050088912
(43) Date of publication of application: 25.06.2008
(73) Proprietor: LG Electronics Inc., Seoul 150-721 (KR)
(72) Inventor: LEE, Wang-Lim, Changwon City, Gyeongsangnam-do 641-761 (KR); HWANG, Hyun-Chul, Jinju City, Gyeongsangnam-do 660-020 (KR); CHOI, Sung-Ho, Daegu City 706-090 (KR)
(74) Representative: Urner, Peter
(86) International application number: PCT/KR2006/003808
(87) International publication number: WO 2007/035069

(56) References cited:
- WO-A1-99/13741
- WO-A2-03/041089
- CN-U- 2 117 166
- JP-A- 01 047 491
- JP-A- 2003 159 314
- KR-Y1- 200 377 915
- US-A- 5 166 528
- US-A1- 2002 122 743

## Description

### [Technical Field]

The present invention relates to a microwave oven, and more particularly, to an ultraviolet (UV) sterilizer for sterilizing articles using ultraviolet rays and a microwave oven including the same.

### [Background Art]

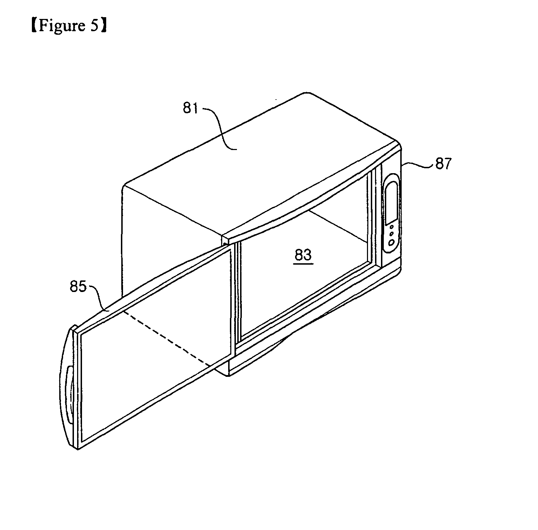

FIG. 5 is a perspective view showing a conventional UV sterilizer.

As shown in this figure, a sterilizing chamber 83 for sterilizing articles thereon is provided in a main body 81 of the sterilizer. The sterilizing chamber 83 is selectively opened or closed by means of a sterilizer door 85 installed pivotally to one end on a front side of the main body 81 in a front and rear direction of the main body 81.

In addition, at least one UV lamp (not shown) is provided on the ceiling of the sterilizing chamber 83. The UV lamp emits UV rays to the sterilizing chamber 83 so as to sterilize articles provided in the sterilizing chamber. Further, a control unit 87 for receiving various signals used to operate the UV sterilizer and displaying a variety of information on the operation of the UV sterilizer is provided to one side of the main body 81.

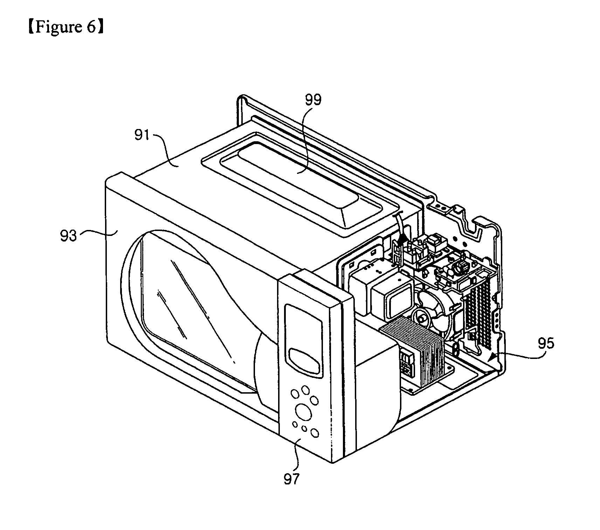

Furthermore, FIG. 6 is a perspective view showing main portions of a conventional microwave oven.

As shown in the figure, a cooking chamber is provided in a cavity 91 of the microwave oven. Food stuffs are cooked in the cooking chamber, and a cooking chamber door 93 is provided to one side of the cavity 91 so as to selectively open or close the cooking chamber. The cooking chamber door 93 is installed to one end on the side of the cavity 91 in a front or rear direction of the cavity 91.

An electric apparatus chamber 95 is provided in a portion of the cavity 91 opposite to the cooking chamber. A variety of electric parts for oscillating microwaves are installed in the electric apparatus chamber 95. In addition, a control unit 97 for receiving various signals used to operate the microwave oven and displaying a variety of information on the operation of the microwave oven is provided to the front of the electric apparatus chamber 95.

In addition, a heater (not shown) is provided to a top surface of the cavity 91 which corresponds to the ceiling of the cooking chamber. The heater is used to provide heat, which is another heat source other than microwaves, and a heater cover 99 is provided to shield the heater.

However, the UV sterilizer and the microwave oven configured as above according to the prior art have the following problems.

In the conventional UV sterilizer, UV rays emitted from the UV lamp are used to sterilize articles received in the sterilizing chamber 83. However, since both the articles and the UV lamp are stationary, there is a problem in that it is not easy to efficiently sterilize articles using UV rays. In particular, in a case where a plurality of articles are stacked one above another in the sterilizing chamber 83, any article placed at a relatively lower position cannot be substantially sterilized by the UV rays emitted from the UV lamp.

In addition, an electronic appliance having various functions, for example, an Internet refrigerator having the existing functions in addition to an Internet function has been recently put into the market. As described above, however, the related art microwave oven has no additional function other than the cooking function. Therefore, there is a problem in that needs of consumers cannot be satisfied.

WO 99/13741 A1 describes a microbial decontamination of food. As shown in figure 1 and 4, a sterilizing unit comprises a treatment cavity defined by an end wall, inner side walls, upper wall and a floor, and an inner wall of a door. UV irradiation is provided within the treatment cavity of the unit by four high intensity UV tubes. The floor houses a turntable having a raised central spindle. The surfaces of the walls and floor are generally highly UV-reflective (for example, they may be constructed from polished zinc plated steel or polished aluminium) in order to enhance the efficacy of irradiation, and to help ensure that all exposed surfaces of the food receive comparable amounts of radiation. Optional door window may have a mirrored surface to maintain the reflectivity of inner wall. An observation window is substantially UV-opaque, and typically will be of safety glass.

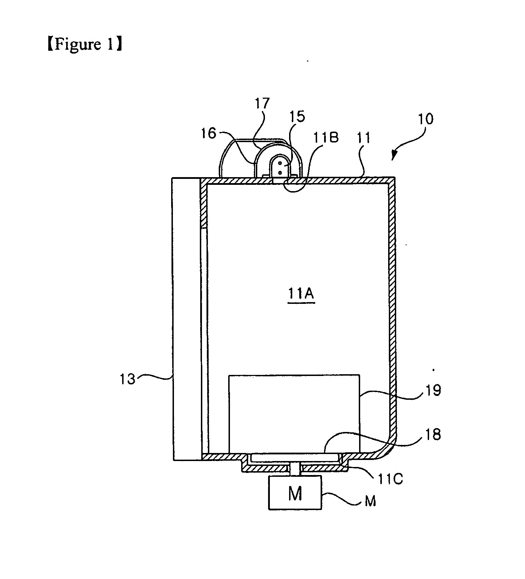

US 2002/0122743 A1 describes an ultraviolet sterilization apparatus and a method for the same. As shown in figure 1, an ultraviolet sterilizer includes a housing unit having a chamber for sterilizing objects. An object to be sterilized is placed in the chamber. The sterilizer is a modified microwave oven and the sterilizing chamber is a food-cooking chamber of the microwave oven. Upon activation of a sterilizing process, the object is irradiated by a UV lamp for a predetermined period o time. Irradiation of the object occurs through an exposure panel located on an interior wall of chamber. The exposure panel may be a fixed-window configuration made of a material that is substantially transparent to a ultraviolet radiation on the UV radiation source side of the chamber, wherein the exposure panel would preferably reflective to microwave radiation on a chamber side of the panel. In contrary, the exposure panel may be made of an opaque material similar to that used in the chamber of a microwave oven, wherein the exposure panel is configured to be open and closed during and after a sterilization process, respectively.

CN 2117166 U describes an ultraviolet disinfecting apparatus. Herein, an UV sterilizing device is provided with an ultraviolet lamp on the inner top surface of the body case. The front side of the device is provided with a glass door for color filtering. The bottom is provided with a control circuit board and an electric motor. The rotating shaft of the electric motor is provided with an object supporting frame, wherein the edge position of which is provided with a small pulley wheel. The lower side surface of the glass door for color filtering is provided with a small magnetic steel, and the corresponding position of the body case is provided with a dry reed pipe.

KR 20-0377915 Y1 describes a microwave oven comprising a cavity having a cooking chamber being closable by means of a first door and a sterilizing chamber located next to the cooking chamber being closable by means of a second door. The sterilizing within the sterilizing chamber is performed by means of UV radiation.

### [Disclosure]

### [Technical Problem]

The present invention is conceived to solve the above problems in the prior art. Accordingly, it is an object of the present invention to provide a UV sterilizer capable of sterilizing articles more efficiently.

It is another object of the present invention to provide a microwave oven including a UV sterilizer with various functions.

### [Technical Solution]

These objects are solved by the microwave oven of claim 1. Further advantages, refinements and embodiments of the invention are described in the respective sub-claims.

According to the present invention, there is provided a microwave oven, comprising: a cavity having a cooking chamber provided to one side thereof to cook food and an electric apparatus chamber provided to an opposite side to the cooking chamber to install a variety of electric parts therein; and an ultraviolet (UV) sterilizer provided in the front of the electric apparatus chamber, comprising: a sterilizer casing having an open side and defining a sterilizing chamber; a sterilizer door installed at one side of the sterilizer casing to selectively open or close the sterilizing chamber; a UV lamp installed at another side of the sterilizer casing to irradiate UV rays into the sterilizing chamber; and a turntable rotably installed on a floor of the sterilizing chamber to place an article to be sterilized onto a top surface thereof. The UV sterilizer further comprises at least one reflection plate installed within the sterilizing chamber to reflect the UV rays emitted from the UV lamp. Further, the UV lamp is installed on the ceiling of the sterilizing chamber, and the reflection plates are installed at lower ends of both sides of the sterilizing chamber, wherein the sterilizer casing is shaped into a polyhedral with rounded edges, and the sterilizing chamber has white inner surfaces to diffusively reflect the UV rays emitted from the UV lamp therein.

Preferably, the sterilizer door is made at least partially of a transparent or translucent material such that the interior of the sterilizing chamber can be viewed from the outside.

Preferably, the UV sterilizer further comprises a lamp cover for shielding the UV lamp and causing the UV rays emitted from the UV lamp to be reflected into the sterilizing chamber.

The sterilizer door is made of the same material as a cooking chamber door which allows the cooking chamber to be selectively opened or closed.

The sterilizing chamber has white inner surfaces to allow the UV rays emitted from the UV lamp to be diffusively reflected therein.

### [Advantageous Effects]

According to the present invention, there are advantages in that the microwave oven with various functions can be provided and articles to be sterilized can be more efficiently sterilized using the microwave oven.

### [Description of Drawings]

FIG. 1 is a side sectional view showing a UV sterilizer.
FIG. 2 is a perspective view showing a microwave oven including the UV sterilizer according to the present invention.
FIG. 3 is a perspective view showing a microwave oven including a UV sterilizer according to the present invention.
FIG. 4 is a sectional view of the UV sterilizer of the microwave oven shown in FIG. 3.
FIG. 5 is a perspective view showing a related art UV sterilizer.
FIG. 6 is a perspective view showing main portions of a related art microwave oven.

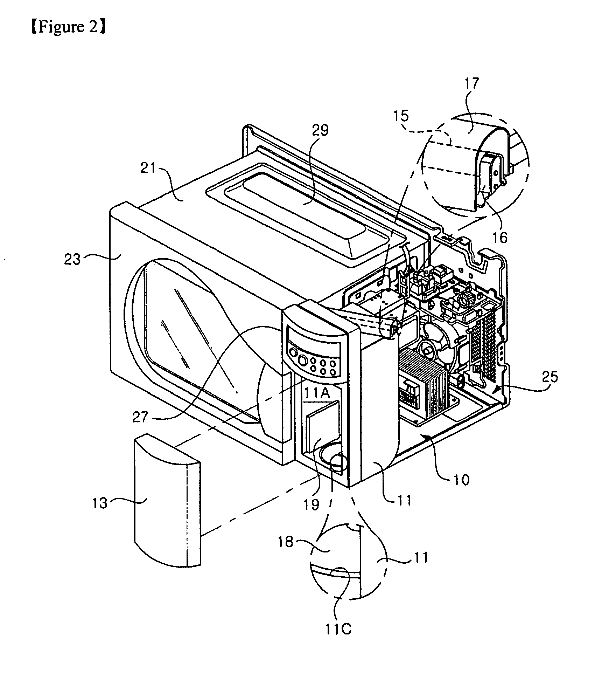

Hereinafter, a microwave oven including an ultraviolet sterilizer according to the present invention will be described in detail with reference to FIG. 1 and FIG. 2.

FIG. 1 is a side sectional view showing an ultraviolet (UV) sterilizer and FIG. 2 is a perspective view showing a microwave oven having the UV sterilizer according to the present invention.

As shown in these figures, an external appearance of the UV sterilizer 10 is defined by a sterilizer casing 11. The sterilizer casing 11 takes the shape of a hexahedron with an open front side. In addition, a sterilizing chamber 11 A is provided in the sterilizer casing 11. Articles can be sterilized in the sterilizing chamber 11A, and a front side of the sterilizing chamber is open toward the outside such that articles to be sterilized can be taken in and out of the chamber through the open front side. Further, a sterilizer door 13 is installed to a portion of the sterilizer casing 11 so as to selectively open or close the sterilizing chamber 11 A.

An opening 11B is formed in a top side of the sterilizer casing 11 corresponding to the ceiling of the sterilizing chamber 11 A. In addition, a UV lamp 15 is provided above the opening 11B. The UV lamp 15 serves to emit UV rays into the sterilizing chamber 11A so as to sterilize articles paced in the chamber. In this embodiment of the present invention, although the UV lamp 15 is provided on the top side of the sterilizer casing 11, the UV lamp 15 may also be provided to a lateral or rear side of the sterilizer casing 11 corresponding to a lateral or rear surface of the sterilizing chamber 11A. Moreover, it is possible to provide a plurality of UV lamps 15 to the sterilizer casing 11.

In addition, a pair of lamp holders 16 are provided to fix the UV lamp 15 to the top side of the sterilizer casing 11. Further, a lamp cover 17 is provided on the top side of the sterilizer casing 11. The lamp cover 17 serves to shield the lamp 15 and also to reflect UV rays irradiated from the UV lamp 15 into the sterilizing chamber 11A.

Meanwhile, the floor of the sterilizing chamber 11A is depressed downward to form an installation groove 11c. In addition, a turntable 18 is installed in the installation groove 11c. The turntable 18 is rotated in a state where articles to be sterilized are placed on a top surface of the turntable. A turntable motor M imparts a rotating force to the turntable 18. The turntable motor M is installed below the sterilizer casing 11.

Further, a pair of reflection plates 19 are provided in the sterilizing chamber 11A. Each of the reflection plates 19 reflects the UV rays irradiated from the UV lamp 15 into the sterilizing chamber 11A such that articles can be sterilized in a more uniform way. The reflection plates 19 are provided at lower ends of both lateral sides of the sterilizing chamber 11A, respectively.

The reflection plates 19 are provided at the lower ends of both lateral sides of the sterilizing chamber 11A. Thus, the reflection plates 19 serve to reflect UV rays toward a lower portion of the article where sterilizing efficiency is relatively lowered since the UV lamp 15 is installed at the top side of the sterilizer casing 11. Thus, the positions of the reflection plates 19 may be determined according to the position of the UV lamp 15.

Although not shown in the figures, various kinds of electric parts, such as a stabilizer, for turning on/off the UV lamp 15 may be provided to one side of the sterilizer casing 11.

Furthermore, the UV sterilizer 10 so configured may be installed to one side of a microwave oven. That is, as shown in FIG. 2, both a cooking chamber and an electric apparatus chamber 25 are provided in a cavity 21 of the microwave oven. The UV sterilizer 10 is also provided in the front of the electric apparatus chamber 25.

At this time, the UV sterilizer 10 is installed in the front of the electric apparatus chamber 25 such that the open front side of the sterilizing chamber 11A is directed toward the front of the cavity 21. In addition, the sterilizer door 13 is installed to a specific portion on the front side of the sterilizer casing 11 so as to selectively open or close the sterilizing chamber 11A.

Reference numerals 23, 27 and 29, which have not yet explained designate a cooking chamber door, a control unit and a heater cover, respectively. The cooking chamber door 23 is pivotally installed to one side of the cavity 21 so as to selectively open or close the cooking chamber. In addition, the sterilizer door 13 is made of the same material as the cooking chamber door 23, so that integrity of the front external appearance of the microwave oven can be obtained. Further, the control unit 27 receives various signals used to operate the microwave oven and the UV sterilizer 10 and displays a variety of information on the operation of the microwave oven and the UV sterilizer 10 to the outside. Moreover, the heater cover 29 serves to shield the heater which becomes a heat source.

Next, a process of sterilizing articles using the UV sterilizer and the microwave oven including the same according to the preferred embodiment of the present invention will be described in more detail.

First, the sterilizer door 13 is opened to allow the sterilizing chamber 11 A to be opened. Further, an article is received in the sterilizing chamber 11A, and the sterilizer door 13 is then closed to shield the sterilizing chamber 11A. At this time, the article received in the sterilizing chamber 11A is placed on the top surface of the turntable 18.

When the article is received in the sterilizing chamber 11A, the control unit 27 is operated to turn on the UV lamp 15. Thus, UV rays are irradiated from the UV lamp 15 into the sterilizing chamber 11A through the opening 11B. In addition, the turntable 18 is also rotated by means of the turntable motor M simultaneously when the UV lamp 15 is turned on.

Therefore, the article is sterilized by means of the UV rays irradiated into the sterilizing chamber 11 A while the article placed on the top surface of the turntable 18 is also being rotated by means of the turntable 18. The UV rays irradiated into the sterilizing chamber 11A are further reflected by the reflection plates 19 such that the article can be sterilized in a more uniform way. In particular, the reflection plates 19 are used to reflect the UV rays irradiated from the UV lamp toward a lower portion of the article which is farthest spaced apart from the UV lamp 15.

Meanwhile, after the article has been sterilized by means of the UV sterilizer 10, the UV lamp 15 is turned off and the turntable 18 is also stopped. Then, the sterilizer door 13 is opened to take the article placed on the top surface of the turntable 18 out of the sterilizing chamber 11A. Thereafter, the sterilizer door 13 is closed to shield the sterilizing chamber 11A.

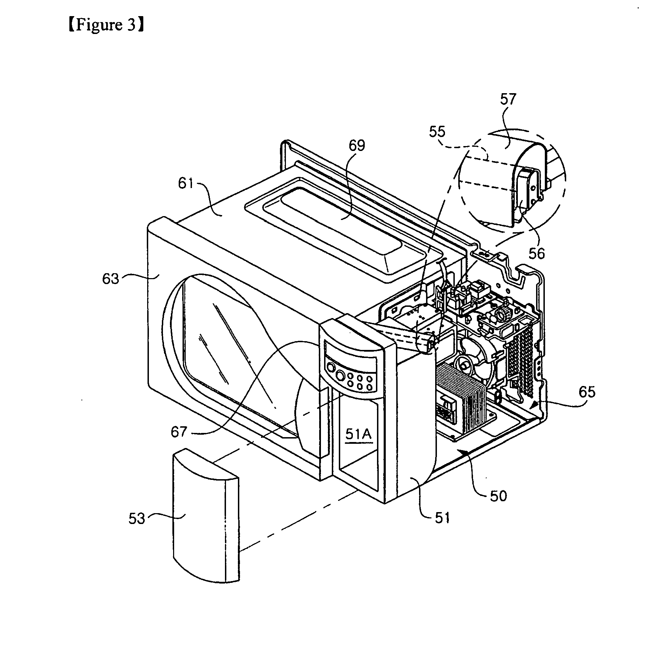

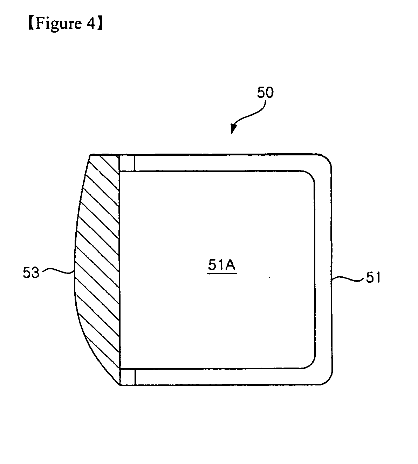

Hereinafter, a microwave oven including a UV sterilizer according to the present invention will be described in detail with reference to FIG. 3 and FIG. 4.

FIG. 3 is a perspective view showing a microwave oven including a UV sterilizer according to the present invention, and FIG. 4 is a sectional view showing the UV sterilizer of the microwave oven shown in FIG. 3.

As shown in these figures, a cooking chamber (not shown) for cooking food therein is provided in a cavity 61 of the microwave oven. In addition, the cooking chamber is selectively opened or closed by a cooking chamber door 63 which is pivotally installed to one side of the cavity 61.

Furthermore, an electric apparatus chamber 65 is provided in the cavity 61 which corresponds to a side opposite to the cooking chamber. In the electric apparatus chamber 65 are installed electric parts for emitting microwaves into the cooking chamber to cook food, a cooling fan for cooling the electric parts, and the like.

Further, a control unit 67 is provided to an upper front end of the electric apparatus chamber 65. A heater cover 69 is also provided to one side of the cavity 61 which corresponds to a top surface of the cooking chamber. The control unit 67 receives various signals used to operate the microwave oven and UV sterilizer 50, and displays a variety of information on the operation of the microwave oven and the UV sterilizer 50, which will be explained later. In addition, the heater cover 69 serves to shield a heater (not shown) which becomes a heat source.

The UV sterilizer 50 is provided in the front of the electric apparatus chamber 65. An external appearance of the UV sterilizer 50 is defined by a sterilizer casing 51. The sterilizer casing 51 takes the shape of a hexahedron with an open front side. In addition, a sterilizing chamber 51 A is defined within the sterilizer casing 51. Articles are taken in and out of the sterilizing chamber 51A through the open front side such that they can be sterilized in the sterilizing chamber 51A.

Further, a sterilizer door 53 is provided to one front side of the cavity 61 so as to selectively open or close the sterilizing chamber 51A. The sterilizer door 53 may be made at least partially or totally of a transparent or translucent material such that a user can view the sterilizing chamber 51A by his/her naked eyes.

Furthermore, as shown in FIG. 4, the sterilizing chamber 51A takes the shape of a hexahedron with open front side and rounded edges corresponding to those of the sterilizer casing 51. In addition, the sterilizing chamber 51 A has white inner surfaces such that the sterilizing chamber 51A can be easily cleaned and UV rays irradiated into the sterilizing chamber can be diffusively reflected.

An opening 51b is formed in a top side of the sterilizer casing 51 corresponding to the ceiling of the sterilizing chamber 51A. In addition, a UV lamp 55 is provided above the opening 51b. The UV lamp 55 serves to emit UV rays into the sterilizing chamber 51 A and then to sterilize articles placed in the chamber.

Further, a pair of lamp holders 56 are provided to fix the UV lamp 55 thereto. A lamp cover 57 is provided to the top side of the sterilizer casing 51 to shield the UV lamp 55 and also to reflect the UV rays irradiated from the UV lamp 55 into the sterilizing chamber 51A.

Moreover, a variety of electric parts, such as a stabilizer (not shown), for turning on/off the UV lamp 55 are provided to one side of the sterilizer casing 51.

Next, a process of sterilizing articles using the UV sterilizer and the microwave oven including the same according to the preferred embodiment of the present invention will be described in more detail.

First, the sterilizer door 53 is opened to allow the sterilizing chamber 51A to be opened. In addition, an article is received into the sterilizing chamber 51 A and the sterilizer door 53 is then closed to shield the sterilizing chamber 51A.

Further, when the article is received in the sterilizing chamber 51A, the control unit 67 is operated to turn on the UV lamp 55. Then, UV rays are irradiated from the UV lamp 55 into the sterilizing chamber 51A through the opening 51b such that the article placed in the sterilizing chamber 51A can be sterilized by means of the UV rays irradiated into the sterilizing chamber 51A.

At this time, since the sterilizing chamber 51A has rounded edges and white inner surfaces, the UV rays emitted from the UV lamp 55 are diffusively reflected within the sterilizing chamber 51A. Thus, the article received in the sterilizing chamber 51A can be sterilized more uniformly by the UV rays.

After the article has been sterilized by the UV sterilizer 50, the sterilizer door 53 is opened to allow the article to be taken out of the sterilizing chamber 51 A. Then, the sterilizer door 53 is closed again to shield the sterilizing chamber 51A.

Meanwhile, while the article is taken into or out of the sterilizing chamber 51A or is sterilized in the sterilizing chamber 51A, the inside of the sterilizing chamber 51A may be stained with foreign substances. As mentioned above, since the sterilizing chamber 51A has the rounded edges, the foreign substances with which the interior of the sterilizing chamber 51A has been stained in the process of sterilizing the articles can be easily cleaned.

### [INDUSTRIAL APPLICABILITY]

According to the present invention, the following advantages can be expected.

First, an article to be sterilized is placed on a top surface of a turntable which is rotably installed to the floor of a sterilizing chamber. Further, the sterilizing chamber has rounded edges and white inner surfaces. Thus, since UV rays are uniformly irradiated into the sterilizing chamber while the article placed on the top surface of the turntable is rotated, uniform sterilization of the article can be expected and the interior of the sterilizing chamber can also be easily cleaned.

Further, since a UV sterilizer is installed to one side of a microwave oven, an article or food can be sterilized or cooked in the microwave oven. Thus, it is possible to produce a microwave oven with various functions, which will be able to meet the needs of the consumers.

Furthermore, the consumers can reduce the costs as compared with when they buy and use individually a microwave oven and a UV sterilizer. Also, a kitchen space can be more efficiently utilized since only one product is installed in the kitchen.

## Claims

1. A microwave oven, comprising:
- a cavity (21, 61) having a cooking chamber provided to one side thereof to cook food and an electric apparatus chamber (25, 65) provided to an opposite side to the cooking chamber to install a variety of electric parts therein; and
- a UV sterilizer (10, 50) comprising:
-- a sterilizer casing (11, 51) having an open side and defining a sterilizing chamber (11A, 51A);
-- a sterilizer door (13, 53) installed at one side of the sterilizer casing (11, 51) to selectively open or close the sterilizing chamber (11A, 51A);
-- a UV lamp (15, 55) installed at another side of the sterilizer casing (11, 51) to irradiate UV rays into the sterilizing chamber (11A, 51A); and
-- a turntable (18) rotatably installed on a floor of the sterilizing chamber (11A, 51A) to place an article to be sterilized onto a top surface thereof;
**characterized in that** the sterilizer (10, 50) further comprises at least one reflection plate (19) installed within the sterilizing chamber (11A, 51A) to reflect the UV rays emitted from the UV lamp (15, 55);
wherein the UV lamp 15, 55) is installed on the ceiling of the sterilizing chamber (11A, 51 A) ; and the reflection plates (19) are installed at lower ends of both sides of the sterilizing chamber (11A, 51A), wherein the sterilizer casing (11, 51) is shaped into a polyhedral with rounded edges, and the sterilizing chamber (11A, 51A) has white inner surfaces to diffusively reflect the UV rays emitted from the UV lamp (15, 55) therein.

2. The microwave oven as claimed in claim 1, the sterilizer door (13, 53) is made at least partially of a transparent or translucent material such that the interior of the sterilizing chamber (11A, 51 A) can be viewed from the outside.

3. The microwave oven as claimed in claim 1, further comprising a lamp cover (17, 57) for shielding the UV lamp (15, 55) and causing the UV rays emitted from the UV lamp (15, 55) to be reflected into the sterilizing chamber (11A, 51A).

## Patentansprüche

1. Mikrowellenherd, der umfasst:
- einen Hohlraum (21, 61), der einen Garraum, der an einer Seite davon vorgesehen ist, um Nahrungsmittel zu garen, und einen Raum für elektrische Betriebsmittel (25, 65), der an der dem Garraum gegenüber liegenden Seite vorgesehen ist, um verschiedene elektrische Teile darin zu installieren, aufweist; und
- einen UV-Sterilisator (10, 50), der umfasst:
-- ein Sterilisatorgehäuse (11, 51), das eine offene Seite aufweist und das einen Sterilisierraum (11A, 51 A) definiert;
-- eine Sterilisatortür (13, 53), die an einer Seite des Sterilisatorgehäuses (11, 51) installiert ist, um wahlweise den Sterilisierraum (11A, 51A) zu öffnen oder zu schlie-βen;
-- eine UV-Lampe (15, 55), die an einer weiteren Seite des Sterilisatorgehäuses (11,51) installiert ist, um UV-Strahlen in den Sterilisierraum (11A, 51A) zu lenken; und
-- eine Drehscheibe (18), die drehbar auf einem Boden des Sterilisierraums (11A, 51A) installiert ist, um einen Gegenstand, der sterilisiert werden soll, auf einer Oberseite davon anzuordnen;
**dadurch gekennzeichnet, dass** der Sterilisator (10, 50) ferner wenigstens eine Reflexionsplatte (19) umfasst, die innerhalb des Sterilisierraums (11A, 51A) installiert ist, um die UV-Strahlen, die von der UV-Lampe (15, 55) emittiert werden, zu reflektieren;
wobei die UV-Lampe (15, 55) an der Decke des Sterilisierraums (11A, 51A) installiert ist; und die Reflexionsplatten (19) an unteren Enden beider Seiten des Sterilisierraums (11A, 51A) installiert sind, wobei das Sterilisatorgehäuse (11, 51) als Polyeder mit abgerundeten Kanten geformt ist und der Sterilisierraum (11A, 51A) weiße innere Oberflächen aufweist, um die UV-Strahlen, die von der UV-Lampe (15, 55) emittiert werden, darin diffus zu reflektieren.

2. Mikrowellenherd nach Anspruch 1, wobei die Sterilisatortür (13, 53) wenigstens teilweise aus einem durchsichtigen oder durchscheinenden Material hergestellt ist, so dass das Innere des Sterilisierraums (11A, 51A) von außen gesehen werden kann.

3. Mikrowellenherd nach Anspruch 1, der ferner eine Leuchtblende (17, 57) umfasst zum Abschirmen der UV-Lampe (15, 55) und Bewirken, dass die UV-Strahlen, die von der UV-Lampe (15, 55) emittiert werden, in den Sterilisierraum (11A, 51A) reflektiert werden.

## Revendications

1. Four à micro-ondes, comprenant :
- une cavité (21,61) possédant une chambre de cuisson sur un côté pour cuire les aliments et une chambre contenant des matériels électriques (25, 65) située sur un côté opposé à la chambre de cuisson pour recevoir divers composants électriques, et
- un stérilisateur UV (10, 50) comprenant :
- une enveloppe de stérilisation (11,51) ayant un côté ouvert et définissant une chambre de stérilisation (11A, 51A) ;
- une porte de stérilisateur (13, 53) installée sur un côté de l'enveloppe de stérilisation (11, 51) pour ouvrir ou fermer de manière sélective la chambre de stérilisation (11A, 51A) ;
- une lampe UV (15, 55) installée sur un autre côté de l'enveloppe de stérilisation (11,51) pour irradier des rayons UV dans la chambre de stérilisation (11A, 51A) ; et
- un plateau tournant (18) monté de manière rotative sur un plancher de la chambre de stérilisation (11A, 51A), afin de placer un article à stériliser sur une surface supérieure de celle-ci ;
**caractérisé en ce que** le stérilisateur (10, 50) comprend en outre au moins une plaque de réflexion (19) installée à l'intérieur de la chambre de stérilisation (11A, 51A), afin de refléter les rayons UV émis par la lampe UV (15, 55) ;
où la lampe UV (15, 55) est installée sur le plafond de la chambre de stérilisation (11A, 51A) ; et les plaques réfléchissantes (19) sont installées aux extrémités inférieures des deux côtés de la chambre de stérilisation (11A, 51A), où l'enveloppe de stérilisation (11, 51) est en forme de polyèdre à bords arrondis et la chambre de stérilisation (11A, 51A) comporte des surfaces internes blanches pour refléter de façon diffuse les rayons UV émis par la lampe UV (15, 55).

2. Four à micro-ondes selon la revendication 1, où la porte du stérilisateur (13, 53) est constituée, au moins partiellement, d'un matériau transparent ou translucide de manière à ce que l'intérieur de la chambre de stérilisation (11A, 51A) puisse être vue de l'extérieur.

3. Four à micro-ondes selon la revendication 1, comprenant en outre un couvercle de la lampe (17, 57) pour faire écran à la lampe UV (15, 55) et entraîner un réfléchissement des rayons UV émis par la lampe UV (15, 55) dans la chambre de stérilisation (11A, 51A).
